# EUROPEAN PATENT SPECIFICATION

(11) **EP 1 363 866 B1**
(45) Date of publication and mention of the grant of the patent: **16.04.2008**
(21) Application number: 02703706.8
(22) Date of filing: 27.02.2002
(51) Int. Cl.: C07C 1/20

(54) **METHOD AND APPARATUS FOR THE PREPARATION OF TRIPTANE AND/OR TRIPTENE**
METHODE UND VORRICHTUNG ZUR HERSTELLUNG VON TRIPTAN UND/ODER TRIPTEN
PROCEDE ET DISPOSITIF PERMETTANT LA PREPARATION DE TRIPTANE ET/OU DE TRIPTENE

(30) Priority: 02.03.2001 GB 0105165; 28.06.2001 GB 0115740
(43) Date of publication of application: 26.11.2003
(73) Proprietor: BP OIL INTERNATIONAL LIMITED, Sunbury-on-Thames, Middlesex TW16 7BP (GB)
(72) Inventor: COOK, Steven, David, Surrey GU15 3AN (GB); COOPER, Jeremy, Bernard, Newick East Sussex BN8 4JX (GB); ELSTNER, Peter, John, Hull HU13 ODP (GB); KAY, Richard, Daniel, Brough East Yorkshire, HU15 2HR (GB); LAUGHTON, Alan, Hedon Hull, HU12 8QY (GB); MORRIS, George, Ernest, Cottingham East Yorkshire, HU16 4BQ (GB); SMITH, Stephen, James, Cottingham East Yorkshire HU16 5LL (GB); SUNLEY, John, Glenn, Cottingham East Yorkshire HU16 4LF (GB)
(74) Representative: Perkins, Nicholas David
(86) International application number: PCT/GB2002/000843
(87) International publication number: WO 2002/070440

(56) References cited:
- US-A- 3 969 427
- US-A- 4 059 647
- US-A- 4 249 031

## Description

This invention relates to a process and apparatus for preparing hydrocarbons, in particular branched chain hydrocarbons.

2,2,3-trimethylbutane, also called triptane, is a branched chain hydrocarbon of high octane number, which can be used in unleaded aviation gasoline and unleaded motor gasoline (see e.g. WO 9822556 and WO 9949003). Most known processes for making it start from expensive starting materials or give very crude mixtures, often containing low proportions of triptane.

The US patent US-A-4 059 647 discloses the production of triptane from methanol or/and dimethylether in the presence of ZnI₂, water by-product being distilled off at the end of the reaction.

A process has now been discovered for making triptane, capable of giving a high yield of triptane and/or a high selectivity to triptane.

According to one aspect, the present invention provides a continuous or semi-continuous process for the production of triptane and/or triptene from methanol and/or dimethyl ether in the presence in a reactor of an effective concentration of catalyst comprising zinc halide and active for the conversion of methanol and/or dimethyl ether to triptane and/or triptene in which process co-produced water is separated from the catalyst as a vapour phase whilst the catalyst is retained in a liquid or solid phase. The separation of water in the vapour phase from catalyst in a liquid/solid phase may be performed in the reactor or in downstream product recovery stages with recycle of catalyst to the reactor.

According to another aspect of the present invention, there is maintained in said reactor an active fonn and an effective concentration of said catalyst, which comprises zinc halide.

The present invention also provides a continuous or semi-continuous process for preparing a product comprising a branched chain hydrocarbon of 7 carbons, which is at least one of triptane and triptene, which process comprises heating an organic feed comprising at least one of methanol and dimethyl ether in the presence of a catalyst comprising zinc halide at a temperature of at least 100°C to produce a mixture comprising (i) methanol and/or dimethyl ether and (ii) a hydrocarbon reaction product comprising said hydrocarbon and in which process the catalyst is maintained in the reactor in an effective form and in an effective concentration and co-produced water is removed from the reactor as the reaction proceeds. The hydrocarbon is then usually separated from the mixture.

The present invention also provides a process for preparing a product comprising a branched chain hydrocarbon of 7 carbons, which is at least one of triptane and triptene, which process comprises heating an organic feed comprising at least one of methanol and dimethyl ether in the presence of a zinc halide at a temperature of at least 100°C to produce a mixture comprising (i) methanol and/or dimethyl ether and (ii) a reaction product comprising said hydrocarbon and co-produced water is removed from the reactor as the reaction proceeds. The said hydrocarbon is then usually separated from said mixture.

The present invention also provides a process for preparing a product which comprises a branched chain hydrocarbon of 7 carbons, which is at least one of triptane and triptene, which comprises heating an organic feed comprising at least one of methanol and dimethyl ether (DME) in the presence of a zinc halide to effect reaction and stopping the reaction before complete conversion of the methanol or dimethyl ether, and recovering the said hydrocarbon and co-produced water is removed from the reactor as the reaction proceeds.

According to the present invention, there is provided a continuous process for the production of triptane and/or triptene, said process comprising :
feeding a reactant stream comprising methanol and/or dimethyl ether into a reactor, and
contacting the reactant stream with a zinc halide catalyst in the reactor to produce a product mixture comprising triptane and/or triptene;
characterised in that the product mixture comprises a first liquid phase and a second liquid phase and co-produced water is removed from the reactor as the reaction proceeds.

Preferably, in the process of the present invention an organic feed comprising at least one of methanol and dimethyl ether (DME) is heated in the presence of a zinc halide to effect reaction and the reaction is stopped before complete conversion of the methanol or dimethyl ether, and the hydrocarbon product is recovered.

Preferably, a mixture of methanol and dimethyl ether is used in the present invention. This has an advantage that the dimethyl ether produces less water than is produced from the methanol. Preferably, some of the dimethyl ether produces methanol in the reactor. If methanol is used in downstream processes of the process, for example in scrubbing gaseous effluent or aiding separations, then there may be a limit to the amount of methanol which can be substituted with dimethyl ether in the process if methanol/dimethyl ether are recycled from the downstream processes to the reactor.

The zinc halide is usually zinc iodide, or bromide or a mixture thereof, but may be zinc chloride or fluoride. Most preferably, the zinc halide is zinc iodide. A suitable salt of zinc halide is preferably anhydrous but it may be used in the form of a solid hydrate.

The catalyst comprising zinc halide may be maintained in an active form and in an effective concentration in the reactor by recycling to the reactor, halide compounds, such as for example hydrogen iodide and/or methyl iodide from downstream product recovery stage(s).

Alternatively, or additionally, there may be introduced to the reactor hydrocarbons such as for example methyl-substituted compounds which stimulate the reaction. Such compounds may comprise methyl substituted compounds selected from the group consisting of aliphatic cyclic compounds, aliphatic heterocyclic compounds, aromatic compounds, heteroaromatic compounds and mixtures thereof. In particular, such compounds may comprise methyl benzenes such as hexamethyl benzene and/or pentamethyl benzene. Preferably, such hydrocarbons are recovered from the reaction composition in downstream product recovery stage(s) and recycled to the reactor.

According to another aspect of the present invention there is provided a continuous or semi-continuous process for the production of triptane and/or triptene which process comprises feeding an organic feed comprising methanol and/or dimethyl ether into a reactor, and
heating the organic feed with the zinc halide catalyst in the reactor to produce a product mixture comprising triptane and/or triptene;
characterised in that the product mixture comprises a first liquid phase, a vapour phase and an optional second liquid phase.

The two liquid phases if present, may be separated by simple separation techniques, such as decantation. This separation step may be carried out continuously or semi-continuously at periodic intervals.

The first liquid phase is typically a hydrophilic phase comprising at least one of the following compounds: water, methanol and dimethyl ether. The first liquid phase may also comprise the zinc halide catalyst. The catalyst may be completely dissolved or there may also be a solid phase present and the catalyst may be partially dissolved in the first liquid phase. Preferably the first liquid phase and any solid phase is retained in the reactor. Alternatively, it may be removed and processed to recover water prior to recycle. Alternatively or additionally, the catalyst may be recovered from the first phase, and optionally, regenerated for re-use.

The second liquid phase if present, is typically a hydrophobic phase comprising the triptane and/or triptene product. Optionally, other oily products, such as by-products of the reaction may also be present in the second phase. Examples of possible by-products include paraffinic and olefinic hydrocarbons, particularly branched paraffinic and olefinic hydrocarbons, and aromatic hydrocarbons. The hydrophobic phase is generally less dense than the hydrophilic phase. The hydrophobic phase may also have dissolved therein one or more of methanol, dimethyl ether, methyl halide (e.g. iodide) and water.

In a preferred embodiment, the second liquid phase if present, is separated from the first liquid phase, and recovered from the reactor. The second liquid phase if present, generally comprises the desired triptane product and/or triptene, which may be converted to the desired triptane product. Triptane is useful in the production of motor and aviation gasoline, especially, unleaded motor and unleaded aviation gasoline. Some or all of the by-product hydrocarbon compounds in the second phase may also be useful in the production of motor and aviation gasoline. Thus, the process of the present invention may further comprise the step of recovering the second liquid phase from the reactor and recovering therefrom hydrocarbons which may comprising triptane and/or triptene. Optionally, at least one motor or aviation gasoline additive may be added to the hydrocarbons recovered from the recovered second liquid phase. The recovered triptane and/or triptene product may be employed as, or as an additive for, a motor or aviation gasoline, preferably, an unleaded motor or aviation gasoline. Preferably the second liquid phase may be purified, for example by distillation, to enhance its concentration of triptane and/or triptene.

In addition to the first liquid phase and optional second liquid phase, the product mixture comprises a vapour phase. The vapour phase may comprise at least one of the following compounds : hydrogen, water vapour, triptane and triptene, methanol and/or dimethyl ether. In a preferred embodiment, water is separated from the catalyst by withdrawing at least some of the vapour phase from the reactor. In a preferred embodiment, the vapour is condensed and is purified, for example, by distillation, to enhance its concentration of triptane and/or triptene. The vapour phase may be purified by condensation and distillation to provide a product water stream clean enough for disposal, a hydrocarbon product comprising triptane and/or triptene and a recycle stream containing unreacted feed components.

In a preferred embodiment the process of the present invention may be performed in a reactor which is suitably an adiabatic reactor or with heat-removal cooling coils which may remove up to 20 % of the heat of reaction. The reactor is provided with a feed inlet through which in use passes con-joined recycle gases, fresh methanol and/or dimethyl ether and recycle methanol. In use, in the reactor the methanol and/or dimethyl ether reacts in the presence of a catalyst comprising zinc halide to produce a mixture comprising water, hydrocarbons (including triptane and/or triptene) and unreacted methanol. In use, the reactor contains a hydrophilic liquid phase comprising the zinc halide catalyst and a vapour phase comprising water and triptane products and optionally a second, upper hydrophobic phase comprising hydrocarbons. Water is removed from the reactor by removing vapour phase from the reactor. Triptane product is recovered from the reactor by removing vapour phase and optionally from liquid phase(s) removed from the reactor. Catalyst components (halide and optionally zinc) removed from the reactor in process streams removed for product recovery are recycled to the reactor to maintain in the reactor an effective concentration of catalyst comprising zinc halide.

The triptane and/or triptene recovered from the reactor (from the vapour phase and optionally from the liquid phase) may be used in the production of motor or aviation gasoline, especially, unleaded motor or unleaded aviation gasoline. At least one motor or aviation gasoline component may be added to the recovered triptane/triptene product. The resulting mixture may be employed as, or as an additive for a motor or aviation gasoline, preferably, an unleaded motor or aviation gasoline.

Methanol and or dimethyl ether present in the recovered vapour phase may be recycled to the reactor.

The contents of the reactor may be mixed. This mixing step may be carried out using any suitable technique, for example, by using a mechanical stirrer and/or by introducing a gas or liquid into the reactor. Any suitable mechanical stirrer may be employed. Preferably the gas which may be bubbled through the reactor to agitate its contents includes recycled unreacted dimethyl ether. Additionally or alternatively, the mixing may be achieved simply as a result of the reactants and/or catalyst being introduced into the reactor.

Although mixing is important for facilitating reaction, it can also inhibit the separation from the first liquid phase of the second liquid phase, if present in the reactor. This problem may be alleviated by reducing the rate of agitation. Preferably, however, at least a portion of the product mixture is at least partially shielded from the full force of the agitation, so that it can separate into at least two liquid phases.

Thus, according to a preferred embodiment, there is provided a continuous or semi-continuous process for the production of triptane and/or triptene, said process comprising:
providing a reaction zone and a separation zone,
feeding an organic feed comprising methanol and/or dimethyl ether into the reaction zone,
contacting the reactant stream with a zinc halide catalyst, to produce a product mixture comprising triptane and/or triptene;
said process being characterised by having at least a portion of the product mixture in the separation zone, so that it can separate into at least two liquid phases.

The reaction zone and separation zone are preferably in fluid communication with each other. The reaction zone and separation zone may be provided in a single piece of apparatus, for example, by using a reactor having a reaction zone and a separation zone.

Alternatively, it is possible to provide the reaction zone and separation zone using separate pieces of apparatus, for example, by coupling a reactor to a separation tank. Multiple reaction zones and/or separation zones may be employed. For example, a reactor having a reaction zone and a separation zone may be coupled to a separate separation tank.

In preferred embodiments of the invention, a reactor having at least one reaction zone and at least one separation zone is employed. Preferably the configuration of the reactor and separation zone will provide good vapour/liquid contact and mixing but at the same time good liquid/liquid separation. For example, the reaction and separation zones may be separated using a grid or perforated plate. In use, the product mixture is allowed to flow freely between the reaction and separation zones through the apertures or perforations in the grid/plate. When the contents of the reactor on one side of the plate/grid is mixed, the reactor contents on the opposite side of the plate/grid is shielded at least in part from the full force of the mixing. Thus, the reactor contents on the opposite side of the grid is in the separation zone, and can separate into at least two phases. The stirrer may be employed in combination with one or more baffles, which may be located in the reactor to enhance the calming effect of the separation zone while maintaining gas/liquid mixing.

The grid or perforated plate may be located in the reactor and placed 0 to 60°, preferably, 0 to 45°, more preferably, 0 to 30° and most preferably, 0 to 15° to the horizontal. In one embodiment, the grid or plate is positioned substantially horizontally. The reactor contents below the grid or plate is agitated, allowing the separation zone to form above the grid or plate. Preferably, the edge(s) of the grid or plate is adjacent to the inner walls of the reactor. The edge(s) may be spaced or in physical engagement with the inner walls of the reactor.

In the embodiment described above, the contents of the reactor is allowed to flow relatively freely through the apertures or perforations of the grid /plate. In an alternative embodiment, the flow of product mixture from the reaction zone to the separation zone may be driven by a mechanical impellor or by the gas lift effect of the bubbles in the reactor. Flow through the separation zone in this mode may be controlled, for example, by positioning a barrier or weir between the reaction zone and separation zone and controlling the driving force across it by controlling the liquid/vapour or liquid/liquid interface levels on either side. Thus, in use, product mixture is allowed to flow from the reaction zone to the separation zone either continuously or at periodic intervals.

In one embodiment of the invention, the contents of the reaction zone are introduced into a further reaction zone before they are introduced into the separation zone. By using more than one reaction zone in this manner, the concentration of unreacted reactants in the product mixture may be reduced to below a threshold value before the product mixture is introduced into the separation zone. This may be advantageous in certain circumstances, as high concentrations of unreacted reactants such as methanol and/or dimethyl ether may inhibit the separation of the product mixture into two liquid phases. Thus, a preferred aspect of the present invention involves the step of ensuring that the water, methanol and/or dimethyl ether concentration of the contents of the separation zone is suitable to ensure that the contents of the separation zone comprises at least two liquid phases.

The process of the present invention may be performed in a reactor provided with heat addition means and/or with heat removal means such as cooling coils. Preferably, such heat removal means may remove up to 20 % of the heat of reaction. The process of the present invention is preferably performed in an adiabatic reactor.

In the process of the present invention the methanol/DME is usually heated with the zinc halide at a temperature of at least 100°C. Preferably, the methanol/DME is heated with the zinc halide at a temperature of 100 to 300 °C, preferably 100-250°C, more preferably 150 to 250 °C. For example, the temperature may be 100-170°C, or 180-230°C. The reaction time is usually 0.1-6 hrs, for example, 0.3-3hrs. Lower temperatures tend to require longer reaction times. Thus, a reaction carried out at 190 to 220°C may require a reaction time of 5-100mins, for example, 10-80 mins. A reaction carried out at 120 to 170°C, on the other hand, may require reaction times of 0.2-2.0hr, for example, 15-60mins. Reaction times or temperatures are usually lower for DME than methanol. The times may be the reaction times in a semi-continuous reaction, or residence time (including average residence time) for continuous processes. The reaction may be monitored for conversion of the methanol or dimethyl ether by periodic sampling in the reaction (for a semi-continuous or continuous process) or in reaction effluent for a continuous process, and then analysis by an appropriate technique e.g. gas liquid chromatography or mass-spectroscopy. The conversion may be 75-95%, but is preferably less than 75%, more preferably, less than 50%. Preferred conversions are 5-75%, for example, 10-50% or more specifically, 20-40%.

The reaction of the present invention is usually performed at elevated pressure such as 5 - 100 barG, preferably 10-100barG, such as 50-100barG. The pressure may be autogeneous, or may by provided also by the presence of an added inert gas, such as nitrogen or argon, and/or preferably by the presence of added hydrogen. Blends of hydrogen with gases inert to the reaction may be used. A mixture of hydrogen and carbon monoxide may be used. The molar ratio of hydrogen to any carbon monoxide present may be at least 5:1. Preferably, the hydrogen in the reaction is in the substantial absence of added carbon monoxide.

The reaction of the present invention may also be performed in the absence or presence of ethylene, propylene and/or butenes such as 2-butene.

The reaction of the present invention is preferably performed in the presence of at least one olefinic initiator or co-reactant, suitably having 2 to 6 carbon atoms. Suitable sources of such olefinic initiators and/or co-reactants are lightly-cracked naphthas such as catalytically cracked spirit and steam cracked spirit.

The ratio of methanol and/or dimethyl ether and water to each other and to the zinc halide catalyst employed in the reaction may vary widely. Since methanol, dimethyl ether and water may be present in one or more liquid phases in the reactor and the vapour phase, and the zinc halide may be present in liquid or solid phase it is convenient to express the concentrations of methanol, dimethyl ether, water and catalyst components as relative weight parts in the reactor excluding any hydrocarbon components or phase. For example, for a total (excluding hydrocarbon components or phase) of 100 weight parts of methanol, dimethyl ether, water and catalyst, the catalyst is preferably greater than 50 parts and less than 99 parts, more preferably greater than 70 parts and less than 95 parts and most preferably greater than 80 parts and less than 90 parts. The water is preferably less than 50 parts and greater than 0, more preferably less than 25 parts and most preferably less than 10 parts. The balance of methanol and dimethyl ether will be to make 100 weight parts. The hydrocarbon components/phase will be additional to this. The ratio of methanol to dimethyl ether may vary from being all dimethyl ether through to all methanol. If preferred, the composition may be chosen to maximise the solubility of the catalyst in the liquid phase or phases at the reaction temperature but operation of the invention is not limited to reactor compositions where no solid phase is present.

The reaction of the present invention may be performed in the absence or presence of an organic diluents. Suitable diluents include liquid alkanes. Such alkanes may be linear, and comprise 9-20, for example, 10-18 carbon atoms. Examples of suitable alkanes include decane or dodecane. As an alternative to linear alkanes, cyclic hydrocarbons may also be employed. Such hydrocarbons include cycloaliphatic hydrocarbons such as cyclohexanes, and liquid aromatic hydrocarbons, such as benzene and its alkyl substituted derivatives, such as toluene or xylene. The diluent may also be an oxygenated hydrocarbon such as a carboxylic acid or carboxylate ester. Suitable esters may be derived from an alkanoic acid of 1-6 carbons and an alkanol of 1-6 carbon atoms. A specific example is of a suitable carboxylic acid is acetic acid. A specific example of a suitable carboxylic ester is methyl acetate. Most preferably, the reaction of the present invention is performed in the presence of aromatic compounds such as methyl benzenes, for example hexamethyl benzene and/or pentamethyl benzene. Preferably, such methyl benzenes are recovered and recycled to the reactor from downstream product separation stage(s) to maintain their standing concentration in the reactor at a steady state. Suitable diluents may include hydrocarbon by-product components separated from the hydrophobic liquid phase or the vapour phase as described above which are then returned to the reactor. Such recycled hydrocarbons may comprise aromatic compounds such as methyl benzenes, for example hexamethyl benzene and/or pentamethyl benzene; naphthenes; olefins and alkanes.

It may be useful to recycle some of the water product as a wash stream to wash out any catalyst dissolved in the second liquid phase.

The process in the absence of added water is especially valuable with methanol as reactant. Water concentration in the reactor must also be limited by removing product water in order to maintain an effective concentration of catalyst. As water is a by-product of the process, it is removed from the reactor as the reaction proceeds suitably to maintain a steady state concentration in the process.

Thus according to the present invention there is provided a process for the production of triptane and/of triptane from methanol and/or dimethyl ether in the presence in a reactor of an effective concentration of catalyst active for the conversion of methanol and/or dimethyl ether to triptane and/or triptene in which process co-produced water is separated from the catalyst as a vapour phase whilst the catalyst is retained in a liquid or solid phase. The separation of water in the vapour phase from catalyst in a liquid/solid phase may be performed in the reactor or in downstream product recovery stages with recycle of catalyst to the reactor.

It may also be possible to remove water from the reactor using techniques such as for example, with desiccants, such as magnesium silicate or molecular sieves e.g. zeolites such as 3A or 13X usually in a non acidic form, e.g. in a metal form when the metal may be an alkali metal (e.g. Na or K) and/or zinc e.g. Na/Zn form.

When the reaction is performed in the absence of water or in the presence of a desiccant, the zinc halide may be supported as an inert support, such as active carbon or another of those listed below as supports for the base, with e.g. 1-10% (wt) zinc halide on the support. The zinc halide may be applied to the support by impregnation and then drying.

The process of the present invention may be performed in the absence or presence of a base, which may be organic. Suitable bases include secondary or tertiary amines, such as a di or trialkyl amine, in which each alkyl has 1-6 carbons, such as ethyl or butyl. Examples of suitable bases include dibutylamine, tributylamine, and aromatic dialkylamine. Where an aromatic amine is employed, the aromatic group may be a substituted phenyl group, such as tolyl or xylyL

The base may also be inorganic. Suitable inorganic bases include metal oxides, hydroxides, alkoxides (e.g. methoxide, ethoxide or t-butoxide), and carboxylates. The metal in the base may be an alkali or alkaline earth metal, for example, Na, K, Ca, and Mg. Preferably, however, the metal is Zn. Thus, particularly useful bases include zinc oxide, zinc methoxide and zinc acetate. If desired the base may be supported on an inert support, e.g. an organic or inorganic one such as silica, alumina, silica/alumina or active carbon; an example is zinc acetate on active carbon. The base may be introduced into the reactor with the methanol and/or dimethyl ether, or as a separate feedstream It is possible to introduce the base into the reactor continuously or at periodic intervals.

The reaction of the present invention may be performed in the presence of hydrogen. Hydrogen may be introduced into the reactor as fresh feed and/or as a component in a recycle stream. Thus, hydrogen may be introduced to the reactor as a separate feed stream, or together with the catalyst and/or one of the other reactants. Alternatively or additionally, the reaction may also be performed in the presence of a hydrogenation catalyst, which may be soluble or insoluble. The catalyst usually comprises a Group VIII metal (of the Periodic Table in Advanced Inorganic Chemistry by FA Cotton and G Wilkinson), for example, Ni, Ru, Rd, Pd, Os, Ir, Pt, and Ru. Preferred examples include catalysts consisting essentially of, or containing ruthenium, nickel and/or palladium. Preferably, a Ru catalyst is employed, optionally, in the presence of Re. The Group VIII metal is, preferably, on an inert support, such as active carbon, for example, charcoal, or alumina, silica or silica/alumina. Amounts of the Group VIII metal in the supported catalyst may be 0.01-30% by weight (expressed as metal): for example, a supported Ni catalyst may comprise 0.01-10% or 10-30% wt Ni.

Preferred catalysts include Ru, Ni or Pd on carbon, Ru on alumina and Ru and Re (in relative wt amounts of 2-6:1) on C. A most preferred catalyst is Ru/C, for example, where the amount of Ru in the catalyst is 0.01-10 wt%.

The reaction of the present invention may be performed in the presence of the hydrogenation catalyst, in the substantial absence of hydrogen when the temperature is below 100°C. However, when the temperature is above 100°C, the reaction is preferably carried out in the presence of hydrogen. Thus, in a semi-continuous reaction involving heating up the reagents to temperature, the hydrogen can be added once the reaction mixture reaches a threshold temperature. Similarly, in a continuous process involving a lower and a higher temperature phase, the hydrogen may be added once the higher temperature phase is reached.

The methanol and/or dimethyl ether fed to the reactor may be introduced continuously or semi-continuously. Preferably, the methanol and/or dimethyl ether is fed to the reactor continuously. The methanol and/or dimethyl ether may be pre-heated before being introduced to the reactor. Suitable pre-heat temperatures range from 30 to 200°C. The process of the present invention may be performed with the methanol/DME heated in the presence of the zinc halide either with direct contact first at the reaction temperature, or with a preheating step in which methanol is heated with zinc chloride at a temperature of less than 100°C, before the heating at the reaction temperature. The reactant feed stream may optionally contain zinc halide catalyst. Alternatively or additionally, the reactor may be pre-loaded with the catalyst. As a further alternative, the catalyst may be introduced into the reactor via a separate feed stream.

The process of the present invention may be performed in a semi-continuous operation, in which the methanol/DME is heated with the zinc halide in a sealed vessel, usually with agitation of the liquid phase and especially with nitrogen and/or hydrogen in the vapour phase. Preferably the vapour phase constitutes less than one third of the volume of the liquid phase especially less than one fifth e.g. 5-30% such as 5-20%. The reactants are usually added into the vessel, which is then pressurised and thereafter heated, the stepwise operation occurring during the heating of the vessel contents up to operating temperature. The progress of the reaction may be monitored as described above. At the end of the reaction period the vessel can be cooled and its contents worked up as described below. Alternatively, the low boiling components of the vessel contents can be distilled off to leave a slurry of solid product comprising zinc halide in methanol/DME and optionally water. The solid product may be ready for reuse in a further batch reaction, after addition of further methanol or DME. The low boiling component distillate may then be purified if desired to recover a fraction rich in triptane and/or triptene.

Preferably, the process of the present invention is performed continuously. The reaction may be in a continuous stirred tank reactor with continuous feed of methanol/DME into an agitated reactor containing the zinc halide or a continuous feed of the methanol/DME and zinc halide either together or separately into the agitated reactor. In both cases, a portion of the product is continuously removed. The process may be performed continuously in a plug flow reactor or a reactor with a moving catalyst bed, passing through a heated zone of a reactor. The mixture made by either continuous process may then be worked up as described.

The recovery of the desired hydrocarbons from the reaction mixture comprising zinc salt may be as follows. If the zinc salt is in solution and there are two liquid layers, an upper hydrophobic one and a lower hydrophilic one (containing the zinc), the two layers may be separated, and the upper layer fractionally distilled to separate any methanol from liquid hydrocarbons. The methanol may be recycled for reuse, and the liquid hydrocarbons may be used as a blending ingredient for gasoline. Alternatively, the liquid hydrocarbons may be purified further by fractional distillation to produce a distillation cut richer in the triptane or triptene. Optionally, the hydrophilic layer may also be distilled to remove methanol or DME, (optionally with some water), which may be recycled for reuse. The residual zinc material in the hydrophilic layer is usually still active in the process and can be recycled for reuse at least once, for example, for 1-4 times. In due course, the zinc halide may become less effective and may require regeneration. This may be done by isolating a zinc containing solid from the hydrophilic layer, for example, by concentrating the layer to leave a slurry of solid. The solid may then be separated, and optionally, extracted with a hydrophobic liquid to extract any organic contaminants present. This extraction process leaves fresh zinc halide which may be reused in the process with methanol or DME. Suitable liquids for the extraction step include alkanes of 5-20 carbons, such as hexane.

If, on the other hand at least some of the zinc salt in the reaction mixture is already in suspension in reaction mixture removed from the reactor, the solid in the suspension may be separated (e.g. by filtration) and extracted as described above to give reusable zinc halide. The liquid separated from the solid in the suspension reaction mixture is usually in the form of two liquid layers, which may be separated and processed as described above.

If the reaction mixture as such, or after separation of insoluble solids, contains only one liquid phase, then the liquid phase may be concentrated by distillation to form a suspension and/or 2 liquid phases each of which can be processed as described above.

In a continuous process, the hydrocarbon reaction product is preferably flash distilled or separated as a liquid layer to leave an hydrophilic layer containing the zinc halide and methanol if the latter were a feed either or both of which can be recycled to the reactor or retained in the reactor, especially after removal of any water.

The methanol or DME is partly converted to a hydrocarbon reaction product, which usually contains alkanes, especially branched alkanes, of 4-8 carbons, in amount of 20-70% preferably, 30-65, more preferably, 40-60%, and higher boiling alkanes and/or aromatics in amount of 80-30%, for example, 70-35% or preferably, 60-40%, all % being expressed on the basis of weight of hydrocarbon reaction product.

The hydrocarbon reaction product usually contains at least 10%, for example, 10-60%, preferably, 20-50% of trimethyl substituted aliphatic hydrocarbon of 7 carbons, in particular triptane optionally mixed with triptene. For example, the hydrocarbon reaction product may comprise 10-50%, preferably, 20-50% triptane and up to 20%, for example, 1-20% preferably, 5-15% triptene.

The hydrocarbon product may also comprise isoalkanes of 4-8 carbons, such as isobutane and/or isopentane. Naphthenes, olefins and aromatics may also be present. These include hexamethyl benzene. A preferred hydrocarbon reaction product is a blend of 20-50% triptane, and 5-15% triptene. The hydrocarbon reaction product may contain a total of 25-60% of trimethyl substituted hydrocarbons of 7 carbons, 35-5% of iso C₄ and C₅ alkanes, and 35-50% of higher boiling aliphatic and aromatic and/or or cycloaliphatic hydrocarbons. Such blends constitute another embodiment of the inventions.

After removal and recycle to the reactor of halide (to maintain an effective concentration of catalyst in the reactor), if desired the hydrocarbon reaction product or blend may be used as such as a blending ingredient in gasolines e.g. unleaded aviation gasolines, but may be distilled first to concentrate the triptane and triptene fractions. Preferably, however, before use for this purpose the hydrocarbon reaction product or blend is hydrogenated to convert triptene into triptane; details of the conditions etc. are described above.

There are a number of preferred ways in which the process may be commercialised, some of which are described below.
(A) The process of the present invention may involve reacting methanol and/or DME and the zinc halide, to form a mixture of methanol and/or DME and the hydrocarbon reaction product. The hydrocarbon reaction product may then be separated from the methanol/DME, from the zinc halide or both, for example, by separation as a different liquid phase or by distillation. The hydrocarbon reaction product may then be separately hydrogenated with hydrogen over a hydrogenation catalyst as described above, for example, at a pressure of 1-10 bar and a temperature of 10-100°C, preferably, 10-50°C. The hydrogenation converts the triptene (and other alkenes) to triptane (and other alkanes).
(B) Alternatively, instead of separating the hydrocarbon reaction product before hydrogenating the product, the hydrogenation can be performed prior to separation. After hydrogenation the triptane and other alkanes can be separated by distillation from the methanol or DME, which can be recycled for reuse.
(C) In yet another alternative, the reaction of the methanol/DME with the zinc halide may be performed at least in part in the presence of hydrogen, either in the presence or absence of the hydrogenation catalyst. At the desired conversion point, the hydrocarbon reaction product can be separated from the zinc halide, and optionally from the methanol/DME, preferably followed by hydrogenation over the catalyst especially if no hydrogenation catalyst were used in the zinc halide reaction step. If desired, the hydrogenation catalyst may be used in both steps. If the hydrocarbon reaction product contains no triptene, then the hydrocarbon reaction product is preferably separated from the zinc halide, and optionally the methanol/DME without any further hydrogenation.

In all these three alternatives (A) to (C), co-product water is separated in a vapour phase from zinc halide catalyst which is retained in a liquid or solid phase.

If desired, the reaction of the present invention may be performed in two steps. This is particularly useful when methanol is employed as the starting material, or when the zinc halide catalyst is of a low activity. The first step may be performed at less than 100°C, to effect conversion of the methanol starting material. The second step may be carried out at more than 100°C, to form at least some C₄-C₈ branched hydrocarbons. If desired, the methanol may be heated at less than 100°C in the presence of a dehydrating agent, prior to the first reaction step. This is particularly useful when the reaction is carried out in a semi-continuous reactor, or when a continuous reactor having only one fixed zinc halide bed is employed.

In a further embodiment, the present invention provides a process for hydrogenation, which comprises hydrogenating with hydrogen and hydrogenation catalyst, a blend comprising triptane and triptene in a weight ratio of 20-50:1-20, such as 20-50:5-15. The blend preferably also comprises isoalkanes of 4-8 carbons, (e.g. in amount of 10-60% of the total of the triptane, triptene and other isoalkanes), and especially also contains alkanes, cycloalkanes and aromatic hydrocarbons of bp greater than 85°C, in weight amount of 35-50% based on the total of blend, isoalkanes and higher boiling components.

Preferably said blend is made by reaction of methanol and/or DME with the zinc halide in the process of the invention with or without separation of the hydrocarbon reaction product.

The invention is illustrated in the following Examples and with reference to the drawings in which Figures 1 and 2 are schematic diagrams of apparatus suitable for carrying out the present invention. Figure 3 shows in graph form analyses on compositions taken during continuous reactions. Figure 4 represents in schematic form apparatus for the continuous production and recovery of triptane.

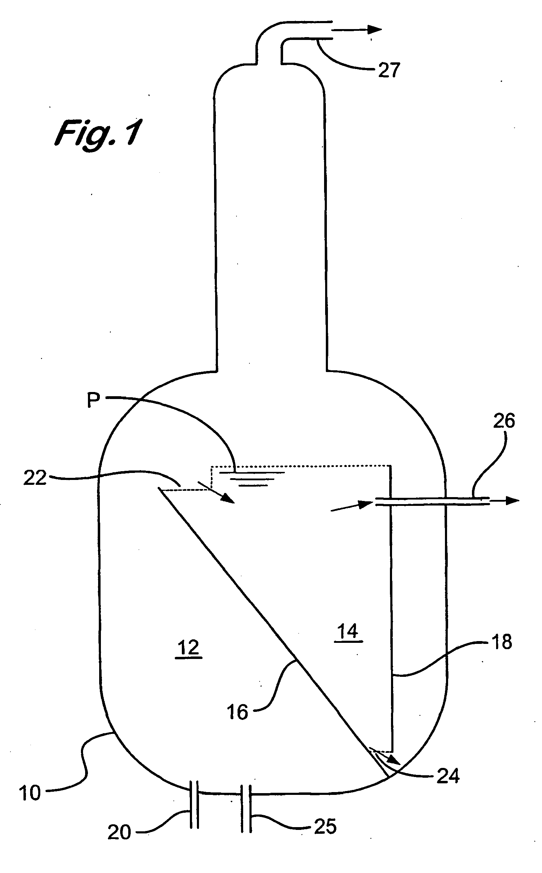

Referring to the drawings, Figure 1 depicts an apparatus suitable for carrying out a first embodiment of the process of the present invention. The apparatus comprises a cylindrical reactor 10, which is divided into a reaction zone 12 and a separation zone 14 by a series of plates 16 and 18. Plate 18 is a baffle positioned vertically on a chord to the reactor's horizontally circular cross-section. Plate 16 is a sloping plate set at an angle to the horizontal. The separation zone 14 is in fluid communication with the reaction zone 12 via an upper inlet 22 and a lower outlet 24.

In operation, a reactant stream comprising methanol is continuously fed through inlet 20 to the reaction zone 12. A gas comprising for example hydrogen and re-cycled dimethyl ether is bubbled through inlet 25 into the reactor 10. The reaction zone 12 is maintained at 200°C and a pressure of 13 bar. Under the reaction conditions, methanol reacts in the presence of catalyst comprising zinc iodide to produce a mixture comprising water, hydrocarbons (comprising triptane) and unreacted methanol.

The reaction zone 12 is in fluid communication with the separation zone 14. Accordingly, once the contents of the reaction zone 12 reaches level P, at least a portion of the product mixture produced in the reaction zone 12 is free to flow into the separation zone 14 via inlet 22. In the separation zone 14, the product mixture is shielded from the agitation caused by the gas stream. Thus, the product mixture in the separation zone 14 is allowed to settle and separate into a light hydrophobic phase, and a heavier hydrophilic phase. The hydrophobic phase comprises hydrocarbons including some of the triptane product, and is continuously recovered from the separation zone via line 26 for further purification (not shown). The hydrophilic phase comprises the unreacted methanol, and is returned to the reaction zone 12 for re-use via outlet 24.

A vapour phase is also present above the hydrophobic phase. This vapour phase also comprises most of the triptane and water products. Thus, the vapour is recovered from the reactor 10 through outlet 27 and condensed (not shown), and purified by distillation (not shown).

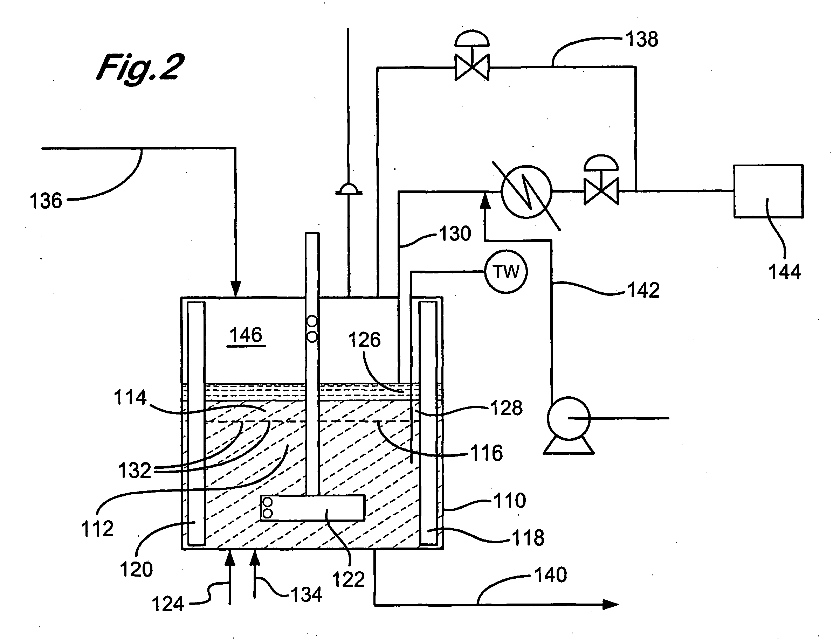

Figure 2 depicts an apparatus suitable for carrying out a second embodiment of the process of the invention. The apparatus comprises a reactor 110, which is divided into a first reaction zone 112 and a separation zone 114 by a horizontal 1 mm grid 116. The reactor is provided with a set of baffle plates 118, 120 and a mechanical stirrer 122. The stirrer 122 extends into the reaction zone 112. The reactor is provided with two inlets 124 and 134 for liquid feeds and an optional inlet 136 for gas. The reactor is also provided with an outlet 140 for first liquid phase, an outlet 130 for gas phase (and second liquid phase, if present) and a second optional gas outlet 138. The outlets 138 and 130 are co-joined and feed to a product pot 144. The gas/second liquid phase outlet 130 is provided with an organic flush (n-hexane) 142.

In operation, a reactant stream comprising methanol and/or dimethyl ether (DME) is continuously fed to the reaction zone 112 through inlet 124. The contents of the reaction zone 112 is agitated by the mechanical stirrer 122. The reaction zone 112 is maintained at 200°C. Under the reaction conditions, methanol and/or DME react in the presence of catalyst comprising zinc iodide to produce a mixture comprising water, hydrocarbons (including triptane) and unreacted methanol.

The contents of the reactor 110 is free to flow between the reaction zone 112 and the separation zone 114 through the apertures 132 of the grid 116. The grid 116, however, shields the product mixture in the separation zone at least in part from the full force of the agitation caused by the stirrer 122. Thus, the product mixture in the separation zone 114 is allowed to settle and separate into a heavier hydrophilic, phase 128 (first liquid phase) and an optional light hydrophobic phase 126 (second liquid phase). The hydrophobic phase 126, if present, comprises heavier hydrocarbons and is continuously recovered from the separation zone via line 130. The heavier liquid phase comprises zinc iodide catalyst, water, methanol etc. The reactor also contains a vapour phase 146, comprising dimethyl ether, water and hydrocarbons including triptane. This vapour phase (comprising water and triptane) is removed through outlet 130 together with second liquid phase if present, or though line 138. The triptane product may be recovered from the vapour phase by condensation distillation (not shown).

Using equipment as shown in Figure 2, (Once Through Unit - OTU) continuous experiments were performed in which dimethyl ether reactant was fed continuously and reaction products (including water and triptane) and unreacted dimethyl ether were removed continuously. Figure 2 illustrates the equipment, with the optional provision to the reactor through inlet 134 of hydrogen iodide solution to maintain in the reactor, an effective concentration of catalyst comprising zinc halide. The reactor (110), internal equipment {agitator (122), baffles (118) and grid plate (116)} and product take-off line (130) were constructed in Hastelloy^{™} B2. The remaining lines were of 316L stainless steel. The reactor was of 300 ml volume; with a normal operating volume of 200 ml. Heating was provided by means of a heating jacket (not shown) with the temperature controlled using an internal thermowell/thermocouple. The reactor pressure was measured by a pressure transmitter on the optional feed gas line and controlled using either the valve on liquid/vapour/gas take-off line 130 and/or the valve on the vapour/gas take-off line 138. A nucleonic source/detector (not shown) was used to indicate the liquid interface in the reactor. A gas dispersion agitator (122) was employed to ensure intimate mixing of the catalyst phase and reactants. The combination of agitator design/rotation speed, grid plate size and baffle configuration ensured mixing in the first reaction zone (112) and separation of the catalyst phase and hydrophobic product phase (126) above the grid plate. The product removed from the reactor was cooled to allow separation from the off gas and collected in a product pot (not shown). The two-phase product was drained and analysed by standard analytical techniques.

### Example 1

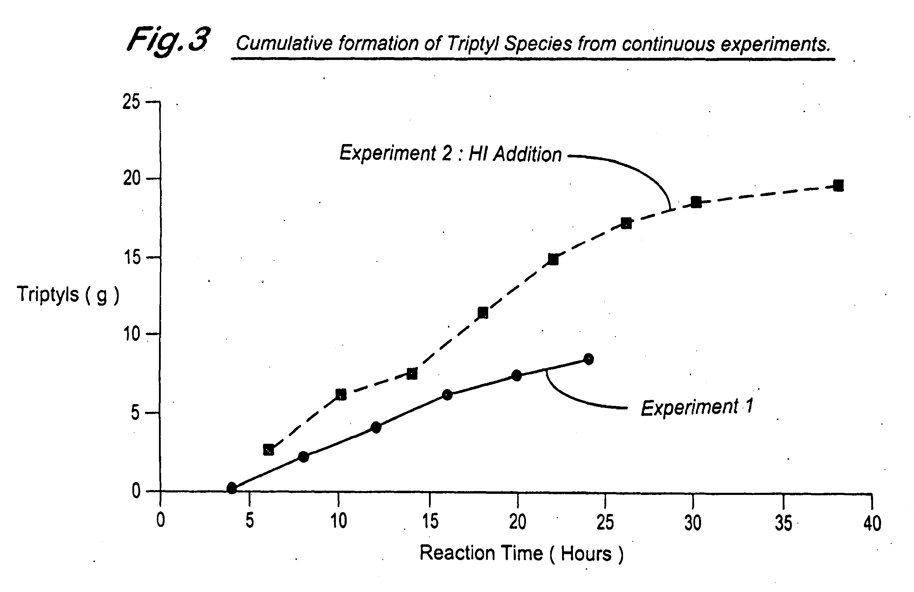

The reactor was charged with 200 ml of a mixture of zinc iodide and methanol in a 1:2 molar ratio. After sealing the reactor, the agitator speed was set to 1000 rpm and the contents heated to 200°C. After leaving under these conditions for 2 hours dimethyl ether feed into the autoclave was commenced at a rate of 53 g/h. Liquid/vapour/gas was removed from the reactor along line 130 to control the pressure and hence maintain liquid contents in the reactor at constant feed rate and temperature. After 24 hours operation the unit was shut down, the feed of dimethyl ether was stopped and the reactor cooled. The off gas from the OTU was primarily unreacted dimethyl ether. Results from this example are given in Tables 1 and 2. The cumulative yield of triptyl products (triptane plus triptene) with time is shown in Figure 3.

### Example 2

This example was carried out using the same procedure as for Example 1, except that aqueous hydroiodic acid was co-fed with the dimethyl ether into the reactor. Also, the dimethyl ether feed rate was 59 g/h. After 44 hours operation dimethyl ether feed was stopped. The off gas from the OTU was primarily unreacted dimethyl ether. Results from this example are given in Tables 1 and 3. The cumulative yield of triptyl products with time is shown in Figure 3. This example demonstrates adding iodide to the system counteracts the loss of methyl iodide from the system and hence maintains in the reactor an effective concentration of catalyst comprising zinc iodide. This improves yield to triptyl species.

**Table 1**

| | Example 1 | Example 2 |
|---|---|---|
| Duration, h | 24 | 38 |
| Average Reactor Pressure, barg | 12.6 | 10.4 |
| Temperature, C | 200 | 200 |
| Dimethyl ether feed, g/h | 53 | 59 |
| HI feed, g/h | 0 | 1.3 |
| Off-gas rate, g/h | 46 | 47 |
| *Aqueous Product* | | |
| Weight, g | 108 | 262 |
| Water, wt% | 59 | 49 |
| Methanol, wt% | 34 | 37 |
| Dimethyl ether, wt% | 5 | 5 |
| Hydrocarbon *Product* | | |
| Weight, g | 54 | 116 |
| Triptane, wt% | 7.5 | 7.1 |
| Triptene, wt% | 8.1 | 9.9 |

**Table 2**

| Example 1 | | | | | |
|---|---|---|---|---|---|
| Elapsed | Triptene | Triptane | Cumulative Make | | |
| Time | Make | Make | Triptene | Triptane | Triptyls |
| h | g | g | g | g | g |
| 4 | 0.1 | 0.1 | 0.1 | 0.1 | 0.1 |
| 8 | 1.1 | 1.0 | 1.2 | 1.0 | 2.2 |
| 12 | 1.0 | 0.9 | 2.2 | 1.9 | 4.1 |
| 16 | 1.1 | 1.0 | 3.3 | 2.9 | 6.2 |
| 20 | 0.7 | 0.6 | 3.9 | 3.5 | 7.5 |
| 24 | 0.5 | 0.5 | 4.4 | 4.0 | 8.4 |

**Table 3**

| Example 2 | | | | | |
|---|---|---|---|---|---|
| Elapsed | Triptene | Triptane | Cumulative | | |
| Time | Make | Make | Triptene | Triptane | Triptyls |
| H | g | g | g | g | g |
| 6 | 1.9 | 0.6 | 1.9 | 0.6 | 2.6 |
| 10 | 2.0 | 1.6 | 4.0 | 2.2 | 6.1 |
| 14 | 0.8 | 0.6 | 4.8 | 2.7 | 7.6 |
| 18 | 2.3 | 1.7 | 7.1 | 4.4 | 11.5 |
| 22 | 1.9 | 1.5 | 9.0 | 5.9 | 14.9 |
| 26 | 1.3 | 1.1 | 10.3 | 7.0 | 17.3 |
| 30 | 0.6 | 0.6 | 10.9 | 7.6 | 18.5 |
| 38 | 0.5 | 0.6 | 11.5 | 8.2 | 19.7 |

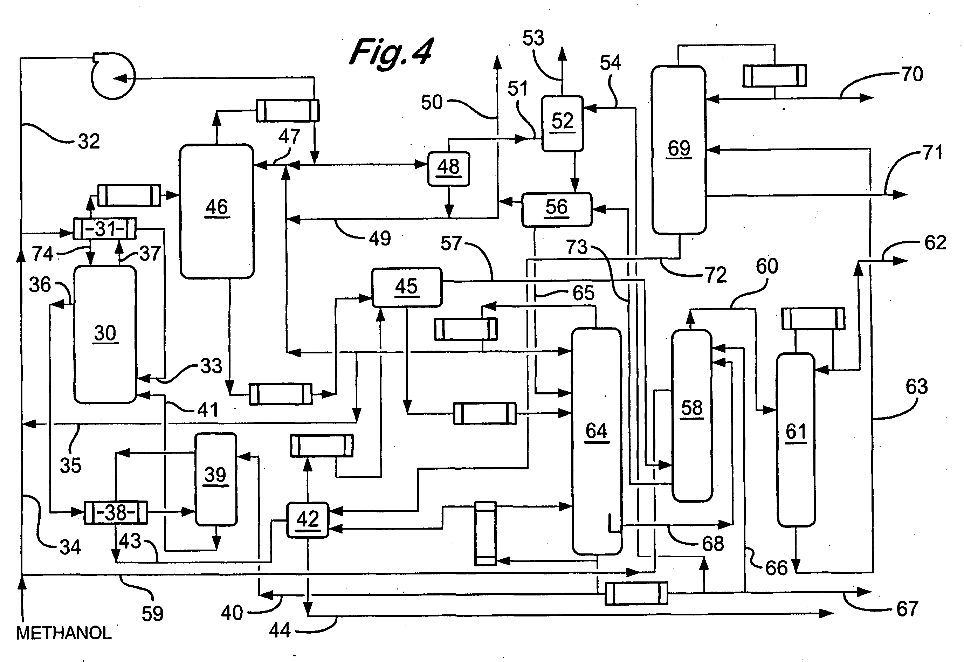

Figure 4 represents in schematic form apparatus for the continuous production and recovery of triptane. Referring to Figure 4, the apparatus comprises a reactor (30) which is suitably an adiabatic reactor or with heat-removal cooling coils (not shown) which may remove up to 20 % of the heat of reaction. The reactor (30) is provided with a feed inlet (33) provided with a feed pre-heater (31) through which in use passes con-joined recycle gases provided by process line (32), fresh methanol and/or dimethyl ether through process line (34) and recycle methanol through process line (35). In use, in the reactor the methanol and/or dimethyl ether reacts in the presence of a catalyst comprising zinc iodide to produce a mixture comprising water, hydrocarbons and unreacted methanol. In use, the reactor contains an upper, hydrophobic liquid phase, a lower, hydrophilic liquid phase comprising the zinc iodide catalyst and a vapour phase comprising water and triptane products.

The reactor is provided with an outlet (36) for hydrophobic liquid phase. The reactor is provided with an outlet (37) for vapour phase which in use, passes through the feed pre-heater. In use, the hydrophobic liquid phase from the reactor passes out of outlet (36) through intercooler (38) to wash column (39) where it is washed with recycle water provided from process line (40). The wash water extracts iodide compounds which are recycled in use to the reactor through line (41) to maintain in the reactor an effective concentration of catalyst comprising zinc iodide. The washed liquid phase is passed through line (43) to flash unit (42) from which is extracted a heavy liquid product along line (44). In use, the vapour from the flash unit (42) is passed to decanter (45).

The vapour phase comprising water and hydrocarbons including triptane is removed from the reactor through feed pre-heater (31) to distillation column (46). Condensed liquid from the pre-heater (31) is returned through process line (74) to the head-space of the reactor as spray to suppress foaming and/or sublimation of hexamethyl benzene. From the head of column (46) is taken recycle gas along line (32) to the reactor. The recycle gas comprises hydrogen, dimethyl ether methyl iodide and large quantities of hydrocarbons. Also from the head of column (46) is taken condensable material which is returned as reflux to the column (46) along line (47) and part of the condensed material is taken to butane flash (48), liquid from which being recycled in part along process line (49) to conjoin the condensed material at the head of the column (46) and part being purged to remove impurities such as dimethyl pentane along line (50). The vapour from butane flasher (48) is passed along process line (51) to a butane wash (52) from the head of which is removed butane product along process line (53). The wash is provided in use along line (54) with recycled water. The base from the butane wash is recycled along process line (55), through oil decanter (56). Decanted oil phase from decanter (56) is conjoined with base from the butane flash (48). The aqueous phase from the decanter is sent elsewhere ("water still" (64)).

In use, the upper phase from decanter (45) is passed along process line (57) to extractor (58) which is a multistage counter-current liquid-liquid extractor and where it is extracted with methanol provided along process line (59), aqueous methanol provided along line (68) and water provided along line (66) to produce a head product which is passed along line (60) to "pure still", distillation column (61) and to produce a base product which is taken along line (73) to decanter (56). From the head of "pure still" (61) is taken a mixed C5/C6 hydrocarbon product along process line (62). From the base of the "pure still" is taken a crude triptane product stream process line (63).

A "water still" distillation column (64) is provided to receive material along process line (65) from oil precipitator (56) and water phase from decanter (45). The base from the "water still" is used in part to provide wash along process line (40) to the liquid phase wash (39), in part to provide wash along process line (54) to "butane wash" unit (52), in part to provide water feed along process line (66) to extractor (58) and the remainder is rejected as waste along process line (67). A liquid side draw is also taken from column (64) along process line (68) as feed to extractor (58).

In use, crude triptane is taken from the base of "pure still" (61) and passed along process line (63) to the "triptane still" distillation column (69). From the head of column (69) is taken pure triptane product along process line (70). From a side draw (71) of the distillation column (69) is taken a C8 hydrocarbon product. A recycle stream is taken from the base of the "triptane still" (69) along process line (72) to the liquid phase flash (42).

The apparatus illustrated in Figure 4 may be used to produce and recover triptane from methanol.

### Further Experiments

In these experiments batch reactions were performed. They are therefore not according to the present invention. However they can be used to illustrate the ways of maintaining an effective concentration of the catalyst comprising zinc halide.

The following experiments illustrate reactions of methanol in the presence of zinc oxide, the effect of zinc oxide on the formation of hydrocarbons and a reaction starting from methyl iodide and zinc oxide. The last experiment illustrates the importance of returning iodide to maintain the catalyst in an active from or in an effective concentration.

A Hastelloy B2 autoclave was charged with reactants in weight parts as shown in Table 4. The autoclave was pressurised with nitrogen to 13 barg, heated to 200 C and maintained at this temperature whilst stirring the reaction mixture. The total reaction time from the start of heating the reaction to starting of cooling the reaction is shown in Table 4. After cooling to ambient temperature the reaction products were recovered and any hydrocarbon layer isolated and analysed. The amount of hydrocarbon layer and the overall selectivity to triptane based on methyl species charged to the autoclave are shown in Table 4

**Table 4**

| Ref | MeOH | Zinc iodide | Methyl iodide | Zinc oxide | Time | Total hydrocarbon | Triptyl selectivity |
|---|---|---|---|---|---|---|---|
| | g | g | g | g | h | g | % |
| G100899 | 14.7 | 75 | 0 | 0 | 2 | 4.1 | 26 |
| D280700 | 15.0 | 75 | 0 | 1 | 2 | 2.9 | 13 |
| D080600 | 15.0 | 42 | 0 | 10 | 2 | 0.0 | 0 |
| G12299 | 0.0 | 0 | 60 | 20 | 4 | 3.7 | 12 |

The following experiments illustrate reaction in the presence of added aromatic hydrocarbons as initiators and therefore as components which can be used to maintain the zinc halide catalyst in an active form or in an effective concentration.

A thick walled glass reaction vessel was charged with zinc iodide, methanol and aromatic hydrocarbon in weight parts as shown in Table 5. The reaction mixture consisted of a clear liquid phase and a solid phase. The reaction vessel was sealed and placed in an oven maintained at the reaction temperature. In these experiments the reaction tubes were not agitated. At the end of the reaction time the reaction tube was removed from the oven and allowed to cool to ambient temperature. After reaction and cooling the reaction mixture consisted of a solid phase and one or two liquid phases. The tube was further cooled, opened and the contents extracted with chloroform (5 parts) and water (3 parts). The two homogeneous liquid phases were analysed and the results are shown in Table 5. The analyses for (MeOH + DME), total hydrocarbons and triptyl species are wt% values derived from normalising the components extracted into the chloroform phase.

**Table 5**

| Ref | Aromatic hydrocarbon | | Zinc iodide | MeOH | Time | TC | MeOH + DME | Total hydrocarbons | Triptyl | Triptyl Sel^{†} |
|---|---|---|---|---|---|---|---|---|---|---|
| | (g) | | (g) | (g) | min | | wt% | wt. %¶ | wt% | % |
| WF37/02 | | | 12 | 2.4 | 74 | 200 | * | * | * | * |
| WF35/02 | HMB | 0.01 | 12.1 | 2.44 | 90 | 200 | 68.33 | 19 | 4.91 | 27 |
| WF35/04 | HMB | 0.10 | 12.1 | 2.62 | 90 | 200 | 10.11 | 72 | 3.82 | 25 |
| WF42/01 | HMB | 0.10 | 10.09 | 2.05 | 160 | 180 | 45.83 | 41.59 | 7.63 | 25 |
| WF34/02 | Mesitylene | 0.34 | 12.09 | 2.41 | 50 | 200 | 11.34 | 72.27 | 7.93 | 19 |
| WF26/03 | TTBB | 1.22 | 8.11 | 1.61 | 90 | 200 | 11.68 | 82.29 | 3.93 | 20 |

| | | | | | | | | | | |
|---|---|---|---|---|---|---|---|---|---|---|
| * No hydrocarbon layer formed during this reaction time ¶ Total hydrocarbon content = 100 - DME (wt%) - MeOH (wt%) - MeI (wt%) † Selectivities exclude aromatic initiator from calculation; HMB-Hexamethyl benzene; TTBB - Tri-tert-butyl benzene | | | | | | | | | | |

The following experiment illustrates reaction in the presence of added reaction product as initiator and therefore as components which can be used to maintain the zinc halide catalyst in an active form or in an effective concentration.
A thick walled glass reaction vessel was charged with zinc iodide (10 parts), methanol (2 parts) and reaction product, being the hydrocarbon product from a continuous reaction such as described in Example 1, (0.1 parts) as shown in Table 5. The reaction mixture consisted of a clear liquid phase and a solid phase. The reaction vessel was sealed and placed in an oven at the reaction temperature. At the end of the reaction time the reaction tube was removed from the oven and allowed to cool to ambient temperature. After reaction and cooling the reaction mixture consisted of a solid phase and one or two liquid phases. The tube was further cooled, opened and the contents extracted with chloroform (5 parts) and water (3 parts). The two homogeneous liquid phases were analysed and the results are shown in Table 6. The analyses for (MeOH + DME), total hydrocarbons and triptyl species are wt% values derived from normalising the components extracted into the chloroform phase.

**Table 6.**

| Ref | Zinc iodide | MeOH | Added Hydro carbon | Time min | TC | MeOH + DME | Total Hydro carbons | Triptyl | Triptyl Sel |
|---|---|---|---|---|---|---|---|---|---|
| | g | g | g | | | wt% | wt% | wt% | % |
| WF37/02 | 12 | 2.4 | 0 | 74 | 200 | * | * | * | * |
| WF54/02 | 10 | 2.0 | 0.1 | 37 | 200 | 8.78 | 74.46 | 13.62 | 18 |

| | | | | | | | | | |
|---|---|---|---|---|---|---|---|---|---|
| * No hydrocarbon layer formed during this reaction time | | | | | | | | | |

The following experiments illustrate reactions in the presence of added alcohols as initiators and therefore as components which can be used to maintain the zinc halide catalyst in an active form or in an effective concentration.

A thick walled glass reaction vessel was charged with zinc iodide, methanol and an alcohol or ether as shown in Table 4. The reaction mixture consisted of a clear liquid phase and a solid phase. The reaction vessel was sealed and placed in an oven at the reaction temperature. At the end of the reaction time the reaction tube was removed from the oven and allowed to cool to ambient temperature. After reaction and cooling the reaction mixture consisted of a solid phase and one or two liquid phases. The tube was further cooled, opened and the contents extracted with chloroform (5 parts) and water (3 parts). The two homogeneous liquid phases were analysed and the results are shown in Table 7. The analyses for (MeOH + DME), total hydrocarbons and triptyl species are wt% values derived from normalising the components extracted into the chloroform phase.

**Table 7.**

| Ref | Alcohol/ Ether | Alcohol/ ether | ZnI₂ | MeOH | H₂O | Time | DME + MeOH | Total Hydrocarbon | Triptyl | Triptyl sel^{†} |
|---|---|---|---|---|---|---|---|---|---|---|
| | g | g | g | g | g | min. | wt% | wt% | wt% | % |
| WF 15/04 | | | 52.7 | 15.3 | | 197 | * | * | * | * |
| WF10/07 | EtOH | 2.64 | 41.03 | 8.79 | 1.01 | 155 | 34 | 56.34 | 13.44 | 24 |
| WF12/01 | IPA | 3.65 | 45.29 | 7.79 | 2.18 | 30 | 20.15 | 75.87 | 6.18 | 8 |
| WF12/04 | IPA | 3.65 | 45.29 | 7.79 | 2.18 | 55 | 9.74 | 88.06 | 11.76 | 14 |
| WF13/01 | 2-BuOH | 2.25 | 42.87 | 9.03 | 1.64 | 30 | 25.15 | 69.83 | 11.09 | 16 |
| WF13/04 | 2-BuOH | 2.25 | 42.87 | 9.03 | 1.64 | 60 | 8.22 | 84.44 | 16.8 | 20 |
| WF9/08 | MTBE | 2.08 | 32.18 | 8.32 | 0 | 35 | 42.16 | 55.56 | 5.30 | 10 |

| | | | | | | | | | | |
|---|---|---|---|---|---|---|---|---|---|---|
| * No hydrocarbon layer had formed during the reaction time † Triptyl selectivities calculated with the exclusion of starting materials derived from the initiator such as the corresponding ether of alkene. | | | | | | | | | | |

## Claims

1. A continuous or semi-continuous process for the production of triptane and/or triptene from methanol and/or dimethyl ether in the presence in a reactor of an effective concentration of catalyst comprising zinc halide and active for the conversion of methanol and/or dimethyl ether to triptane and/or triptene in which process co-produced water is removed from the reactor as the reaction proceeds and is separated from the catalyst as a vapour phase whilst the catalyst is retained in a liquid or solid phase.

2. A process as claimed in claim 1 in which the separation of water in the vapour phase from catalyst in a liquid/solid phase is performed in the reactor.

3. A process as claimed in claim 1 in which the separation of water in the vapour phase from catalyst in a liquid/solid phase is performed in downstream product recovery stages with recycle of catalyst to the reactor.

4. A process as claimed in any preceding claim in which the catalyst is maintained in the reactor in an active form and in an effective concentration by recycling to the reactor, halide compounds, from downstream product recovery stage(s).

5. A process as claimed in claim 4, which process comprises heating an organic feed comprising at least one of methanol and dimethyl ether in the presence of a catalyst comprising zinc halide at a temperature of at least 100°C to produce a mixture comprising (i) methanol and/or dimethyl ether and (ii) a hydrocarbon reaction product comprising said hydrocarbon and in which process the catalyst is maintained in the reactor in an effective form and in an effective concentration.

6. A process for preparing a product comprising a branched chain hydrocarbon of 7 carbons, which is at least one of triptane and triptene, which process comprises heating an organic feed comprising at least one of methanol and dimethyl ether in the presence of a zinc halide at a temperature of at least 100°C to produce a mixture comprising (i) methanol and/or dimethyl ether and (ii) a reaction product comprising said hydrocarbon and in which process co-produced water is removed from the reactor as the reaction proceeds.

7. A continuous or semi-continuous process as claimed in claim 6 for preparing a product comprising a branched chain hydrocarbon of 7 carbons, which is at least one of triptane and triptene, which process comprises heating an organic feed comprising at least one of methanol and dimethyl ether in the presence of a catalyst comprising zinc halide at a temperature of at least 100°C to produce a mixture comprising (i) methanol and/or dimethyl ether and (ii) a hydrocarbon reaction product comprising said hydrocarbon and in which process the catalyst is maintained in the reactor in an effective form and in an effective concentration and co-produced water is removed from the reactor as the reaction proceeds.

8. A process for preparing a product which comprises a branched chain hydrocarbon of 7 carbons, which is at least one of triptane and triptene, which comprises heating an organic feed comprising at least one of methanol and dimethyl ether (DME) in the presence of a zinc halide to effect reaction and stopping the reaction before complete conversion of the methanol or dimethyl ether, and recovering the said hydrocarbon and in which process co-produced water is removed from the reactor as the reaction proceeds.

9. A continuous process for the production of triptane and/or triptene, said process comprising:
feeding a reactant stream comprising methanol and/or dimethyl ether into a reactor, and
contacting the reactant stream with a zinc halide catalyst in the reactor to produce a product mixture comprising triptane and/or triptene;
**characterised in that** the product mixture comprises a first liquid phase and a second liquid phase and co-produced water is removed from the reactor as the reaction proceeds.

10. A process as claimed in any one of the preceding claims in which a mixture of methanol and dimethyl ether is used.

11. A process as claimed in any one of claims 4 to 10 in which the zinc halide is selected from the group consisting of zinc iodide, zinc bromide and mixtures thereof.

12. A process as claimed in any one of the preceding claims in which there is introduced to the reactor hydrocarbons which comprise methyl substituted compounds selected from the group consisting of aliphatic cyclic compounds, aliphatic heterocyclic compounds, aromatic compounds, heteroaromatic compounds and mixtures thereof, preferably they comprise methyl benzenes such as hexamethyl benzene and/or pentamethyl benzene, to stimulate the reaction.

13. A process as claimed in claim 12 in which said hydrocarbons are recovered from the reaction composition in downstream product recovery stage(s) and recycled to the reactor.

14. A process as claimed in any one of claims 4 to 13 in which the organic feed comprising at least one of methanol and dimethyl ether is heated in the presence of a zinc halide in the reactor at a temperature of 100 to 300°C, preferably 100 to 250°C, more preferably 150 to 250°C.

15. A process as claimed in any one of the preceding claims in which the reaction is performed in the presence of at least one olefinic initiator or co-reactant, suitably having 2 to 6 carbon atoms.

16. A process as claimed in claim 15 in which the olefinic initiator or co-reactant is sourced from a lightly-cracked naphtha such as catalytically cracked spirit or steam cracked spirit.

17. A process as claimed in any one of the preceding claims in which the reaction is performed in the presence of hydrogen and/or in the presence of a hydrogenation catalyst.

18. A process as claimed in any one of claims 4 to 17 which process comprises feeding an organic feed comprising methanol and/or dimethyl ether into a reactor, and heating the organic feed with the zinc halide catalyst in the reactor to produce a product mixture comprising triptane and/or triptene; **characterised in that** the product mixture comprises a first liquid phase, a vapour phase and an optional second liquid phase.

19. A process as claimed in claim 18 in which the liquid product comprises first and second liquid phases, the second liquid phase being a hydrophobic phase comprising triptane and/or triptene product.

20. A process as claimed in claim 19 comprising:
providing a reaction zone and a separation zone;
feeding an organic feed comprising methanol and/or dimethyl ether into the reaction zone;
contacting the reactant stream with a zinc halide catalyst, to produce a product mixture comprising triptane and/or triptene;
said process being **characterised by** having at least a portion of the product mixture in the separation zone, so that it can separate into at least two liquid phases.

21. A process as claimed in claim 20 in which the reaction zone and separation zone are provided in a reactor having a reaction zone and a separation zone.

22. A process as claimed in claim 21 in which the reaction and separation zones are separated using a grid or perforate plate.

23. A process as claimed in any one of claims 19 to 22 which further comprises the step of recovering the second liquid phase comprising triptane and/or triptene from the reactor.

24. A process as claimed in claim 23 which further comprises purifying the second liquid phase, for example, by distillation, to enhance its concentration of triptane and/or triptene.

25. A process as claimed in any one of claims 18 to 24 in which the vapour phase is condensed and is purified, for example by distillation, to enhance its concentration of triptane and/or triptene.

26. A process as claimed in claim 25 in which the vapour phase is purified by condensation and distillation to provide a product water stream clean enough for disposal, a hydrocarbon product comprising triptane and/or triptene and a recycle stream containing unreacted feed components.

27. A process as claimed in any one of the preceding claims in which at least one motor or aviation gasoline additive is added to the recovered triptane and/or triptene product.

28. A process as claimed in any one of the preceding claims in which the reaction is performed in the presence of aromatic compounds such as methyl benzenes, for example hexamethyl benzene and/or pentamethyl benzene.

29. A process as claimed in claim 28 in which the methyl benzenes are recovered and recycled to the reactor from downstream product separation stage(s) to maintain their standing concentration in the reactor at a steady state.

30. A process as claimed in claim 19 in which the reaction is performed in the presence of hydrocarbon by-product components separated from the hydrophobic liquid phase or the vapour phase which are then returned to the reactor.

31. A process as claimed in claim 30 in which the recycled hydrocarbons comprise aromatic compounds such as methyl benzenes, for example hexamethyl benzene and/or pentamethyl benzene; naphthenes; olefins and alkanes.

## Patentansprüche

1. Kontinuierliches oder halb-kontinuierliches Verfahren zur Herstellung von Triptan und/oder Tripten aus Methanol und/oder Dimethylether in Gegenwart einer wirksamen Konzentration an Katalysator, der Zinkhalogenid umfaßt und für die Umwandlung von Methanol und/oder Dimethylether in Triptan und/oder Tripten aktiv ist, in einem Reaktor, wobei in dem Verfahren gleichzeitig erzeugtes Wasser im Verlauf der Reaktion aus dem Reaktor entfernt und als eine Dampfphase von dem Katalysator abgetrennt wird, während der Katalysator in einer flüssigen oder festen Phase bleibt.

2. Verfahren nach Anspruch 1, wobei das Abtrennen des Wassers in der Dampfphase von dem Katalysator in einer flüssigen/festen Phase in dem Reaktor durchgeführt wird.

3. Verfahren nach Anspruch 1, wobei das Abtrennen des Wassers in der Dampfphase von dem Katalysator in einer flüssigen/festen Phase in Downstream-Produktrückgewinnungsstufen unter Rückführung des Katalysators in den Reaktor durchgeführt wird.

4. Verfahren nach einem der vorhergehenden Ansprüche, wobei der Katalysator in dem Reaktor in aktiver Form und in wirksamer Konzentration gehalten wird, indem Halogenidverbindungen aus der/den Downstream-Produktrückgewinnungsstufe(n) in den Reaktor zurückgeführt werden.

5. Verfahren nach Anspruch 4, wobei das Verfahren das Erwärmen einer organischen Speisung, umfassend mindestens eines von Methanol und Dimethylether, in Gegenwart eines Katalysators, umfassend Zinkhalogenid, bei einer Temperatur von mindestens 100 °C zur Herstellung eines Gemisches, umfassend (i) Methanol und/oder Dimethylether und (ii) ein Kohlenwasserstoff Reaktionsprodukt, umfassend den Kohlenwasserstoff, umfaßt, und wobei in dem Verfahren der Katalysator in dem Reaktor in wirksamer Form und in einer wirksamen Konzentration gehalten wird.

6. Verfahren zur Herstellung eines Produktes, umfassend einen verzweigtkettigen Kohlenwasserstoff mit 7 Kohlenstoffen, das mindestens eines von Triptan und Tripten ist, wobei das Verfahren das Erwärmen einer organischen Speisung, umfassend mindestens eines von Methanol und Dimethylether, in Gegenwart eines Zinkhalogenids bei einer Temperatur von mindestens 100 °C zur Herstellung eines Gemisches, umfassend (i) Methanol und/oder Dimethylether und (ii) ein Reaktionsprodukt, umfassend den Kohlenwasserstoff, umfaßt, und wobei in dem Verfahren gleichzeitig erzeugtes Wasser im Verlauf der Reaktion aus dem Reaktor entfernt wird.

7. Kontinuierliches oder halb-kontinuierliches Verfahren nach Anspruch 6 zur Herstellung eines Produktes, umfassend einen verzweigtkettigen Kohlenwasserstoff mit 7 Kohlenstoffen, das mindestens eines von Triptan und Tripten ist, wobei das Verfahren das Erwärmen einer organischen Speisung, umfassend mindestens eines von Methanol und Dimethylether, in Gegenwart eines Katalysators, umfassend Zinkhalogenid, bei einer Temperatur von mindestens 100 °C zur Herstellung eines Gemisches, umfassend (i) Methanol und/oder Dimethylether und (ii) ein Kohlenwasserstoff-Reaktionsprodukt, umfassend den Kohlenwasserstoff, umfaßt, und wobei in dem Verfahren der Katalysator in dem Reaktor in wirksamer Form und in einer wirksamen Konzentration gehalten wird und gleichzeitig erzeugtes Wasser im Verlauf der Reaktion aus dem Reaktor entfernt wird.

8. Verfahren zur Herstellung eines Produktes, das einen verzweigtkettigen Kohlenwasserstoff mit 7 Kohlenstoffen umfaßt, das mindestens eines von Triptan und Tripten ist, welches das Erwärmen einer organischen Speisung, umfassend mindestens eines von Methanol und Dimethylether (DME), in Gegenwart eines Zinkhalogenids zur Herbeiführung einer Reaktion und das Stoppen der Reaktion vor der vollständigen Umwandlung des Methanols oder Dimethylethers und das Rückgewinnen des Kohlenwasserstoffes umfaßt, und wobei in dem Verfahren gleichzeitig erzeugtes Wasser im Verlauf der Reaktion aus dem Reaktor entfernt wird.

9. Kontinuierliches Verfahren zur Herstellung von Triptan und/oder Tripten, wobei das Verfahren
das Einspeisen eines Reaktantenstroms, umfassend Methanol und/oder Dimethylether, in einen Reaktor, und
das Kontaktieren des Reaktantenstroms mit einem Zinkhalogenid-Katalysator in dem Reaktor zur Herstellung eines Produktgemisches, umfassend Triptan und/oder Tripten, umfaßt;
**dadurch gekennzeichnet, daß** das Produktgemisch eine erste flüssige Phase und eine zweite flüssige Phase umfaßt und gleichzeitig erzeugtes Wasser im Verlauf der Reaktion aus dem Reaktor entfernt wird.

10. Verfahren nach einem der vorhergehenden Ansprüche, wobei ein Gemisch aus Methanol und Dimethylether verwendet wird.

11. Verfahren nach einem der Ansprüche 4 bis 10, wobei das Zinkhalogenid aus der Gruppe ausgewählt ist, bestehend aus Zinkiodid, Zinkbromid und Gemischen davon.

12. Verfahren nach einem der vorhergehenden Ansprüche, wobei Kohlenwasserstoffe in den Reaktor eingeführt werden, die Methyl-substituierte Verbindungen, ausgewählt aus der Gruppe, bestehend aus aliphatischen cyclischen Verbindungen, aliphatischen heterocyclischen Verbindungen, aromatischen Verbindungen, heteroaromatischen Verbindungen und Gemischen davon, und bevorzugt Methylbenzole wie Hexamethylbenzol und/oder Pentamethylbenzol, umfassen, um die Reaktion zu stimulieren.

13. Verfahren nach Anspruch 12, wobei die Kohlenwasserstoffe aus der Reaktionszusammensetzung in der/den Downstream-Produktrückgewinnungsstufe(n) rückgewonnen und in den Reaktor zurückgeführt werden.

14. Verfahren nach einem der Ansprüche 4 bis 13, wobei die organische Speisung, die mindestens eines von Methanol und Dimethylether umfaßt, in Gegenwart eines Zinkhalogenids in dem Reaktor bei einer Temperatur von 100 bis 300 °C, bevorzugt 100 bis 250 °C, stärker bevorzugt 150 bis 250 °C, erwärmt wird.

15. Verfahren nach einem der vorhergehenden Ansprüche, wobei die Reaktion in Gegenwart mindestens eines olefinischen Initiators oder Co-Reaktanten, geeigneterweise mit 2 bis 6 Kohlenstoffatomen, durchgeführt wird.

16. Verfahren nach Anspruch 15, wobei der olefinische Initiator oder Co-Reaktant aus leicht gecracktem Rohbenzin wie katalytisch gecracktem Benzin oder dampfgecracktem Benzin stammt.

17. Verfahren nach einem der vorhergehenden Ansprüche, wobei die Reaktion in Gegenwart von Wasserstoff und/oder in Gegenwart eines Hydrierungskatalysators durchgeführt wird.

18. Verfahren nach einem der Ansprüche 4 bis 17, wobei das Verfahren
das Einspeisen einer organischen Speisung, umfassend Methanol und/oder Dimethylether, in einen Reaktor, und
das Erwärmen der organischen Speisung mit dem Zinkhalogenid-Katalysator in dem Reaktor zur Herstellung eines Produktgemisches, umfassend Triptan und/oder Tripten, umfaßt; **dadurch gekennzeichnet, daß** das Produktgemisch eine erste flüssige Phase, eine Dampfphase und eine optionale zweite flüssige Phase umfaßt.

19. Verfahren nach Anspruch 18, wobei das flüssige Produkt eine erste und zweite flüssige Phase umfaßt, wobei die zweite flüssige Phase eine hydrophobe Phase ist, die das Triptan- und/oder Triptenprodukt umfaßt.

20. Verfahren nach Anspruch 19, umfassend:
das Bereitstellen einer Reaktionszone und einer Trennzone;
das Einspeisen einer organischen Speisung, umfassend Methanol und/oder Dimethylether, in die Reaktionszone;
das Kontaktieren des Reaktantenstroms mit einem Zinkhalogenid-Katalysator zur Herstellung eines Produktgemisches, umfassend Triptan und/oder Tripten;
wobei das Verfahren **dadurch gekennzeichnet ist, daß** sich mindestens ein Teil des Produktgemisches in der Trennzone befindet, so daß es sich in mindestens zwei flüssige Phasen trennen kann.

21. Verfahren nach Anspruch 20, wobei die Reaktionszone und die Trennzone in einem Reaktor mit einer Reaktionszone und einer Trennzone bereitgestellt werden.

22. Verfahren nach Anspruch 21, wobei die Reaktions- und Trennzone unter Verwendung eines Gitters oder einer perforierten Platte getrennt sind.

23. Verfahren nach einem der Ansprüche 19 bis 22, das ferner den Schritt der Rückgewinnung der zweiten flüssigen Phase, umfassend Triptan und/oder Tripten, aus dem Reaktor umfaßt.

24. Verfahren nach Anspruch 23, das ferner die Reinigung der zweiten flüssigen Phase, zum Beispiel durch Destillation, zur Erhöhung ihrer Konzentration an Triptan und/oder Tripten umfaßt.

25. Verfahren nach einem der Ansprüche 18 bis 24, wobei die Dampfphase kondensiert und beispielsweise durch Destillation gereinigt wird, um ihre Konzentration an Triptan und/oder Tripten zu erhöhen.

26. Verfahren nach Anspruch 25, wobei die Dampfphase durch Kondensation und Destillation gereinigt wird, um einen Produktwasserstrom, der zur Entsorgung rein genug ist, ein Kohlenwasserstoffprodukt, umfassend Triptan und/oder Tripten, und einen Rücklaufstrom, der nicht umgesetzte Speisungskomponenten enthält, bereitzustellen.

27. Verfahren nach einem der vorhergehenden Ansprüche, wobei dem rückgewonnen Triptan- und/oder Triptenprodukt mindestens ein Fahr- oder Flugbenzinadditiv zugegeben wird.

28. Verfahren nach einem der vorhergehenden Ansprüche, wobei die Reaktion in Gegenwart aromatischer Verbindungen wie Methylbenzolen, zum Beispiel Hexamethylbenzol und/oder Pentamethylbenzol, durchgeführt wird.

29. Verfahren nach Anspruch 28, wobei die Methylbenzole rückgewonnen und in den Reaktor aus der/den Downstream-Produktrückgewinnungsstufe(n) rückgeführt werden, um ihre bestehende Konzentration in dem Reaktor im stationären Zustand zu halten.

30. Verfahren nach Anspruch 19, wobei die Reaktion in Gegenwart von Kohlenwasserstoffnebenproduktkomponenten durchgeführt wird, die aus der hydrophoben flüssigen Phase oder der Dampfphase abgetrennt wurden, die dann in den Reaktor rückgeführt werden.

31. Verfahren nach Anspruch 30, wobei die rückgeführten Kohlenwasserstoffe aromatische Verbindungen wie Methylbenzole, zum Beispiel Hexamethylbenzol und/oder Pentamethylbenzol, Naphthene, Olefine und Alkane umfassen.

## Revendications

1. Procédé continu ou semi-continu permettant la production de triptane et/ou de triptène à partir du méthanol et/ou du diméthyléther en présence dans un réacteur d'une concentration efficace d'un catalyseur comprenant un halogénure de zinc et actif pour la conversion du méthanol et/ou du diméthyléther en triptane et/ou en triptène, dans lequel procédé, l'eau co-produite est extraite du réacteur au fur et à mesure que la réaction progresse et est séparée du catalyseur sous forme d'une phase vapeur tandis que le catalyseur est retenu dans une phase liquide ou solide.

2. Procédé selon la revendication 1, dans lequel la séparation de l'eau dans la phase vapeur à partir du catalyseur dans une phase liquide/solide, s'effectue dans le réacteur.

3. Procédé selon la revendication 1, dans lequel la séparation de l'eau dans la phase vapeur à partir du catalyseur dans une phase liquide/solide s'effectue dans des étages de récupération de produits en aval avec recyclage du catalyseur vers le réacteur.

4. Procédé selon l'une quelconque des revendications précédentes, dans lequel le catalyseur est maintenu dans le réacteur dans une forme active et dans une concentration efficace en recyclant vers le réacteur des composés halogénures provenant de l'étage (des étages) de récupération de produits en aval.

5. Procédé selon la revendication 4, lequel procédé comprend le chauffage d'une charge organique comprenant au moins un élément parmi le méthanol et le diméthyléther en présence d'un catalyseur comprenant un halogénure de zinc à une température d'au moins 100°C pour produire un mélange comprenant (i) du méthanol et/ou du diméthyléther et (ii) un produit de réaction d'hydrocarbure comprenant ledit hydrocarbure et dans lequel procédé, le catalyseur est maintenu dans le réacteur dans une forme efficace et dans une concentration efficace.

6. Procédé permettant la préparation d'un produit comprenant un hydrocarbure à chaîne ramifiée à 7 atomes de carbone, qui est au moins un élément parmi le triptane et le triptène, lequel procédé comprend le chauffage d'une charge organique comprenant au moins un élément parmi le méthanol et le diméthyléther en présence d'un halogénure de zinc à une température d'au moins 100°C pour produire un mélange comprenant (i) du méthanol et/ou du diméthyléther et (ii) un produit de réaction comprenant ledit hydrocarbure et dans lequel procédé, l'eau co-produite est extraite du réacteur au fur et à mesure que la réaction progresse.

7. Procédé continu ou semi-continu selon la revendication 6, permettant la préparation d'un produit comprenant un hydrocarbure à chaîne ramifiée à 7 atomes de carbone, qui est au moins un élément parmi le triptane et le triptène, lequel procédé comprend le chauffage d'une charge organique comprenant au moins un élément parmi le méthanol et le diméthyléther en présence d'un catalyseur comprenant un halogénure de zinc à une température d'au moins 100°C pour produire un mélange comprenant (i) du méthanol et/ou du diméthyléther et (ii) un produit de réaction d'hydrocarbure comprenant ledit hydrocarbure et dans lequel procédé, le catalyseur est maintenu dans le réacteur dans une forme efficace et dans une concentration efficace et l'eau co-produite est extraite du réacteur au fur et à mesure que la réaction progresse.

8. Procédé permettant la préparation d'un produit qui comprend un hydrocarbure à chaîne ramifiée à 7 atomes de carbone, qui est au moins un élément parmi le triptane et le triptène, qui comprend le chauffage d'une charge organique comprenant au moins un élément parmi le méthanol et le diméthyléther (DME) en présence d'un halogénure de zinc pour effectuer la réaction et l'arrêt de la réaction avant la conversion complète du méthanol ou du diméthyléther, et la récupération dudit hydrocarbure et dans lequel procédé l'eau co-produite est extraite du réacteur au fur et à mesure que la réaction progresse.

9. Procédé continu permettant la production de triptane et/ou de triptène, ledit procédé comprenant :
le chargement d'un courant de réactif comprenant du méthanol et/ou du diméthyléther dans un réacteur, et la mise en contact du courant de réactif avec un catalyseur à halogénure de zinc dans le réacteur pour produire un mélange de produits comprenant du triptane et/ou du triptène ;
**caractérisé en ce que** le mélange de produits comprend une première phase liquide et une seconde phase liquide et l'eau co-produite est extraite du réacteur au fur et à mesure que la réaction progresse.

10. Procédé selon l'une quelconque des revendications précédentes, dans lequel on utilise un mélange de méthanol et de diméthyléther.

11. Procédé selon l'une quelconque des revendications 4 à 10, dans lequel l'halogénure de zinc est choisi dans le groupe constitué par l'iodure de zinc, le bromure de zinc et un de leurs mélanges.

12. Procédé selon l'une quelconque des revendications précédentes, dans lequel on introduit dans le réacteur des hydrocarbures qui comprennent des composés substitués sur le méthyle choisis dans le groupe constitué par des composés cycliques aliphatiques, des composés hétérocycliques aliphatiques, des composés aromatiques, des composés hétéroaromatiques et un de leurs mélanges, ils comprennent de préférence des méthylbenzènes comme l'hexaméthylbenzène et/ou le pentaméthylbenzène, pour stimuler la réaction.

13. Procédé selon la revendication 12, dans lequel lesdits hydrocarbures sont récupérés dans la composition de la réaction dans un (des) étage(s) de récupération de produits en aval et recyclés vers le réacteur.

14. Procédé selon l'une quelconque des revendications 4 à 13, dans lequel la charge organique comprenant au moins un élément parmi le méthanol et le diméthyléther est chauffée en présence d'un halogénure de zinc dans le réacteur à une température de 100 à 300°C, de préférence de 100 à 250°C, plus préférablement de 150 à 250°C.

15. Procédé selon l'une quelconque des revendications précédentes, dans lequel la réaction s'effectue en présence d'au moins un initiateur ou co-réactif oléfinique, ayant de préférence de 2 à 6 atomes de carbone.

16. Procédé selon la revendication 15, dans lequel l'initiateur ou le co-réactif oléfinique provient d'un naphta légèrement craqué comme de l'essence craquée catalytiquement ou de l'essence craquée à la vapeur.

17. Procédé selon l'une quelconque des revendications précédentes, dans lequel la réaction s'effectue en présence d'hydrogène et/ou en présence d'un catalyseur d'hydrogénation.

18. Procédé selon l'une quelconque des revendications 4 à 17, lequel procédé comprend le chargement d'une charge organique comprenant du méthanol et/ou du diméthyléther dans un réacteur, et le chauffage de la charge organique avec le catalyseur à halogénure de zinc dans le réacteur pour produire un mélange de produits comprenant du triptane et/ou du triptène ; **caractérisé en ce que** le mélange de produits comprend une première phase liquide, une phase vapeur et une seconde phase liquide optionnelle.

19. Procédé selon la revendication 18, dans lequel le produit liquide comprend des première et seconde phases liquides, la seconde phase liquide étant une phase hydrophobe comprenant du produit de triptane et/ou de triptène.

20. Procédé selon la revendication 19, comprenant :
le fait de prévoir une zone de réaction et une zone de séparation ;
le chargement d'une charge organique comprenant du méthanol et/ou du diméthyléther dans la zone de réaction ;
la mise en contact du courant de réactif avec un catalyseur à halogénure de zinc, pour produire un mélange de produits comprenant du triptane et/ou du triptène ;
ledit procédé étant **caractérisé par** le fait d'avoir au moins une partie du mélange de produits dans la zone de séparation, de sorte qu'il puisse se séparer en au moins deux phases liquides.

21. Procédé selon la revendication 20, dans lequel la zone de réaction et la zone de séparation sont prévues dans un réacteur ayant une zone de réaction et une zone de séparation.

22. Procédé selon la revendication 21, dans lequel les zones de réaction et de séparation sont séparées en utilisant une grille ou une plaque perforée.

23. Procédé selon l'une quelconque des revendications 19 à 22, qui comprend en outre l'étape consistant à récupérer la seconde phase liquide comprenant le triptane et/ou le triptène dans le réacteur.

24. Procédé selon la revendication 23, qui comprend en outre la purification de la seconde phase liquide, par exemple, par distillation, pour renforcer sa concentration de triptane et/ou de triptène.

25. Procédé selon l'une quelconque des revendications 18 à 24, dans lequel la phase vapeur est condensée et est purifiée, par exemple par distillation, pour renforcer sa concentration de triptane et/ou de triptène.

26. Procédé selon la revendication 25, dans lequel la phase vapeur est purifiée par condensation et distillation pour fournir un courant aqueux de produit suffisamment propre pour être rejeté, un produit d'hydrocarbure comprenant du triptane et/ou du triptène et un courant de recyclage contenant des composants de charge n'ayant pas réagi.

27. Procédé selon l'une quelconque des revendications précédentes, dans lequel au moins un additif d'essence automobile ou avion est ajouté au produit de triptane et/ou de triptène récupéré.

28. Procédé selon l'une quelconque des revendications précédentes, dans lequel la réaction s'effectue en présence de composés aromatiques comme des méthylbenzènes, par exemple de l'hexaméthylbenzène et/ou du pentaméthylbenzène.

29. Procédé selon la revendication 28, dans lequel les méthylbenzènes sont récupérés et recyclés vers le réacteur à partir d'un étage (d'étages) de séparation de produits en aval pour maintenir leur concentration permanente dans le réacteur dans un état stable.

30. Procédé selon la revendication 19, dans lequel la réaction s'effectue en présence de composants de produits dérivés d'hydrocarbures séparés de la phase liquide hydrophobe ou de la phase vapeur qui sont ensuite renvoyés vers le réacteur.

31. Procédé selon la revendication 30, dans lequel les hydrocarbures recyclés comprennent des composés aromatiques comme des méthylbenzènes, par exemple de l'hexaméthylbenzène et/ou du pentaméthylbenzène ; des naphtènes ; des oléfines et des alcanes.
